(19) **Europäisches Patentamt**

**European Patent Office**

**Office européen des brevets**

(11) **EP 1 269 837 A2**

(12) **EUROPEAN PATENT APPLICATION**

(43) Date of publication:
02.01.2003 Bulletin 2003/01

(51) Int Cl.⁷: **A01K 67/027**, C07K 14/435, A61K 39/35, A23C 9/20

(21) Application number: 02252702.2

(22) Date of filing: 17.04.2002

(84) Designated Contracting States:
**AT BE CH CY DE DK ES FI FR GB GR IE IT LI LU MC NL PT SE TR**
Designated Extension States:
**AL LT LV MK RO SI**

(30) Priority: **08.06.2001 US 877160**

(71) Applicant: **TaiMont Biotech, Inc.**
**Tainan County (TW)**

(72) Inventors:
• **Ching-Hsaing, Hsu**
**Taibo City, Chiayi County (TW)**

• **Cheng, Winston T.K.**
**Taipei 10764 (TW)**
• **Chen, Chuan-Mu Dr.**
**Dept. of Zoology,Life. Sc. Col.**
**402, Taichung City, Taiwan, R.O.C. (TW)**

(74) Representative: **Lunt, Mark George Francis et al**
**Harrison Goddard Foote,**
**Fountain Precinct,**
**Leopold Street**
**Sheffield S1 2QD (GB)**

(54) **Allergen-containing milk for allergy treatment**

(57) A milk composition that includes a heterologous non-milk allergen can be administered to a subject to suppress allergen-specific IgE production in the subject

EP 1 269 837 A2

SEQUENCE LISTING

<110> Ching-Hsaing, Hsu
      Cheng, Winston T. K.

<120> ALLERGEN-CONTAINING MILK FOR ALLERGY
      TREATMENT

<130> P71896EP

<140> 09/877,160
<141> 2001-06-08

<160> 10

<170> FastSEQ for Windows Version 4.0

<210> 1
<211> 132
<212> PRT
<213> Dermatophagoides pteronyssinus

<400> 1
Met Lys Phe Ile Ile Ala Phe Phe Val Ala Thr Leu Ala Val Met Thr
 1               5                  10                  15
Val Ser Gly Glu Asp Lys Lys His Asp Tyr Gln Asn Glu Phe Asp Phe
            20                  25                  30
Leu Leu Met Glu Arg Ile His Glu Gln Ile Lys Lys Gly Glu Leu Ala
            35                  40                  45
Leu Phe Tyr Leu Gln Glu Gln Ile Asn His Phe Glu Glu Lys Pro Thr
            50                  55                  60
Lys Glu Met Lys Asp Lys Ile Val Ala Glu Met Asp Thr Ile Ile Ala
65                  70                  75                  80
Met Ile Asp Gly Val Arg Gly Val Leu Asp Arg Leu Met Gln Arg Lys
                85                  90                  95
Asp Leu Asp Ile Phe Glu Gln Tyr Asn Leu Glu Met Leu Lys Lys Ser
            100                 105                 110
Gly Asp Ile Leu Glu Arg Asp Leu Lys Lys Glu Glu Ala Arg Val Lys
            115                 120                 125
Asn Ile Glu Val
    130

<210> 2
<211> 320
<212> PRT
<213> Dermatophagoides pteronyssinus

<400> 2
Met Lys Ile Val Leu Ala Ile Ala Ser Leu Leu Ala Leu Ser Ala Val
 1               5                  10                  15
Tyr Ala Arg Pro Ser Ser Ile Lys Thr Phe Glu Glu Tyr Lys Lys Ala
            20                  25                  30
Phe Asn Lys Ser Tyr Ala Thr Phe Glu Asp Glu Glu Ala Ala Arg Lys
            35                  40                  45
Asn Phe Leu Glu Ser Val Lys Tyr Val Gln Ser Asn Gly Gly Ala Ile
            50                  55                  60

```
Asn His Leu Ser Asp Leu Ser Leu Asp Glu Phe Lys Asn Arg Phe Leu
65                  70                  75                  80
Met Ser Ala Glu Ala Phe Glu His Leu Lys Thr Gln Phe Asp Leu Asn
                85                  90                  95
Ala Glu Thr Asn Ala Cys Ser Ile Asn Gly Asn Ala Pro Ala Glu Ile
            100                 105                 110
Asp Leu Arg Gln Met Arg Thr Val Thr Pro Ile Arg Met Gln Gly Gly
            115                 120                 125
Cys Gly Ser Cys Trp Ala Phe Ser Gly Val Ala Ala Thr Glu Ser Ala
        130                 135                 140
Tyr Leu Ala Tyr Arg Asn Gln Ser Leu Asp Leu Ala Glu Gln Glu Leu
145                 150                 155                 160
Val Asp Cys Ala Ser Gln His Gly Cys His Gly Asp Thr Ile Pro Arg
                165                 170                 175
Gly Ile Glu Tyr Ile Gln His Asn Gly Val Val Gln Glu Ser Tyr Tyr
            180                 185                 190
Arg Tyr Val Ala Arg Glu Gln Ser Cys Arg Arg Pro Asn Ala Gln Arg
            195                 200                 205
Phe Gly Ile Ser Asn Tyr Cys Gln Ile Tyr Pro Pro Asn Val Asn Lys
        210                 215                 220
Ile Arg Glu Ala Leu Ala Gln Thr His Ser Ala Ile Ala Val Ile Ile
225                 230                 235                 240
Gly Ile Lys Asp Leu Asp Ala Phe Arg His Tyr Asp Gly Arg Thr Ile
                245                 250                 255
Ile Gln Arg Asp Asn Gly Tyr Gln Pro Asn Tyr His Ala Val Asn Ile
            260                 265                 270
Val Gly Tyr Ser Asn Ala Gln Gly Val Asp Tyr Trp Ile Val Arg Asn
        275                 280                 285
Ser Trp Asp Thr Asn Trp Gly Asp Asn Gly Tyr Gly Tyr Phe Ala Ala
        290                 295                 300
Asn Ile Asp Leu Met Met Ile Glu Glu Tyr Pro Tyr Val Val Ile Leu
305                 310                 315                 320
```

```
<210> 3
<211> 146
<212> PRT
<213> Dermatophagoides pteronyssinus

<400> 3
Met Met Tyr Lys Ile Leu Cys Leu Ser Leu Leu Val Ala Ala Val Ala
1               5                   10                  15
Arg Asp Gln Val Asp Val Lys Asp Cys Ala Asn His Glu Ile Lys Lys
            20                  25                  30
Val Leu Val Pro Gly Cys His Gly Ser Glu Pro Cys Ile Ile His Arg
            35                  40                  45
Gly Lys Pro Phe Gln Leu Glu Ala Val Phe Glu Ala Asn Gln Asn Thr
        50                  55                  60
Lys Thr Ala Lys Ile Glu Ile Lys Ala Ser Ile Asp Gly Leu Glu Val
65                  70                  75                  80
Asp Val Pro Gly Ile Asp Pro Asn Ala Cys His Tyr Met Lys Cys Pro
                85                  90                  95
Leu Val Lys Gly Gln Gln Tyr Asp Ile Lys Tyr Thr Trp Asn Val Pro
            100                 105                 110
Lys Ile Ala Pro Lys Ser Glu Asn Val Val Val Thr Val Lys Val Met
        115                 120                 125
Gly Asp Asp Gly Val Leu Ala Cys Ala Ile Ala Thr His Ala Lys Ile
        130                 135                 140
```

```
Arg Asp
145


<210> 4
<211> 21
<212> DNA
<213> Artificial Sequence


<220>
<223> oligonucleotide for PCR


<400> 4
tcgcgtagaa tcgattcatg t                                        21


<210> 5
<211> 21
<212> DNA
<213> Artificial Sequence


<220>
<223> oligonucleotide for PCR


<400> 5
tacgcgtagc ctgggtggca t                                        21


<210> 6
<211> 20
<212> DNA
<213> Artificial Sequence


<220>
<223> oligonucleotide for PCR


<400> 6
gctcgagcat gaaattcatc                                          20


<210> 7
<211> 23
<212> DNA
<213> Artificial Sequence


<220>
<223> oligonucleotide for PCR


<400> 7
actcgagtga tgaaggcaac aag                                      23


<210> 8
<211> 20
<212> DNA
<213> Artificial Sequence


<220>
<223> oligonucleotide for PCR


<400> 8
ctctcttgtc atcctcttcc                                          20
```

<210> 9
<211> 23
<212> DNA
<213> Artificial Sequence

<220>
<223> oligonucleotide for PCR

<400> 9
actcgagtga tgaaggcaag aag    23

<210> 10
<211> 624
<212> DNA
<213> Dermatophagoides pteronyssinus

<220>
<221> CDS
<222> (62)...(457)

<400> 10
gtgaccccat ttcagaatct tgacggctaa ccatcactag tgaattcgcg gccgctcgag    60
c atg aaa ttc atc att gct ttc ttt gtt gcc act ttg gca gtt atg act    109
  Met Lys Phe Ile Ile Ala Phe Phe Val Ala Thr Leu Ala Val Met Thr
   1          5               10             15

gtt tca ggt gaa gat aaa aaa cat gat tat caa aat gaa ttt gat ttc    157
Val Ser Gly Glu Asp Lys Lys His Asp Tyr Gln Asn Glu Phe Asp Phe
           20              25             30

tta ttg atg gaa cgt att cat gaa caa att aaa aaa ggt gaa ctt gca    205
Leu Leu Met Glu Arg Ile His Glu Gln Ile Lys Lys Gly Glu Leu Ala
        35              40             45

ttg ttc tat ctt caa gaa cag att aat cat ttt gaa gaa aaa cca aca    253
Leu Phe Tyr Leu Gln Glu Gln Ile Asn His Phe Glu Glu Lys Pro Thr
      50              55             60

aaa gaa atg aaa gat aaa att gta gcc gaa atg gat acc att att gct    301
Lys Glu Met Lys Asp Lys Ile Val Ala Glu Met Asp Thr Ile Ile Ala
 65             70             75             80

atg atc gat ggt gta cgt ggt gta ctt gat cgt ctt atg caa cgt aaa    349
Met Ile Asp Gly Val Arg Gly Val Leu Asp Arg Leu Met Gln Arg Lys
           85              90             95

gat tta gat att ttt gaa caa tat aat ctt gaa atg ctc aaa aaa tct    397
Asp Leu Asp Ile Phe Glu Gln Tyr Asn Leu Glu Met Leu Lys Lys Ser
          100            105           110

ggt gat att ttg gaa cgt gat ttg aaa aaa gaa gaa gca cgt gtt aaa    445
Gly Asp Ile Leu Glu Arg Asp Leu Lys Lys Glu Glu Ala Arg Val Lys
      115              120          125

aat att gaa gtt aagctttgaa ttcagatccg ttaccaacta cctagactgg    497
Asn Ile Glu Val

```
    130

    attcgtgaca atatgcggcc gtgatatcta cgtatgatca gcctcgactg tgccttcttg    557
    ttgccttcat cactcgagtc tagagggccc gtttaaaccc gctgatcagc ctcgactgtg    617
    ccttcta                                                               624
```

**Description**

Background of the Invention

Asthma, a chronic inflammatory disease of the airways, currently affects over 155 million people worldwide and its prevalence is increasing. It affects approximately one in four children (25%), one in seven adolescents, and one in ten adults. People with asthma collectively have more than 100 million days of restricted activity and 470,000 hospitalizations annually. More than 5,000 people die of asthma annually.

The association between mite allergy and diseases such as asthma, allergic rhinitis, and atopic dermatitis is well known. The predominant mite species in Taiwan, Australia, and Western Europe is *Dermatophagoides pteronyssinus*, whereas *Dermatophagoides farinae* is predominant in many areas of the United States, Japan, and continental Europe.

IgE in the serum of patients allergic to mites reacts with a variety of components. As determined by Western blotting, this interaction can result in specific binding of one to two to over fourteen different mite species. The Der p 1, Der p 2 and Der p 5 allergens react with about 80% of allergic sera, often in high titers, and have been characterized by cDNA cloning, as well as by binding to monoclonal antibodies and other immunochemical procedures.

Allergen immunotherapy, the predominant available treatment, deals with causes of allergic diseases by modifying or down-regulating the immune response. Properly used, allergen immunotherapy is both effective and relatively safe, and is a necessary part of the management of allergic disease.

Allergen immunotherapy is typically administered by parenteral injection of allergens. Other routes of administration, including oral, sublingual, nasal and bronchial routes, have also been tried. Oral and sublingual immunotherapy given to experimental animals indicates that ingestion of allergens may induce tolerance. In addition, sublingual immunotherapy has been shown to reduce allergic symptoms and/or medication needs. The efficacy is similar to subcutaneous injection immunotherapy performed in children.

In immunotherapy, recombinant allergens with authentic tertiary structure display efficacy and safety profiles similar to those of naturally occurring allergens.

Summary of the Invention

The invention is based on the discovery that the milk of a transgenic mammal that contains a non-milk, heterologous allergen can induce immunological tolerance against the allergen in animal models. In addition, this tolerance reduced hyperreactivity and inflammation, two symptoms associated with exposure to allergens.

Accordingly, the invention includes a transgenic mammal whose genetic composition includes a nucleic acid sequence that includes (1) a coding sequence encoding an allergen and (2) a heterologous promoter operably linked to the coding sequence. The promoter directs expression of the coding sequence in a mammary cell. The allergen can be an allergen from a common dust mite, such as *Dermatophagoides pteronyssinus, D. farinae, D. microceras, Tyrophagus putesentiae, Lepidoglyphus domesticus, L. destructor, Acarus siro, Euroglyphus maynei,* and *Biomia tropicalis*; or other airborne allergen (aeroallergen) such as pollens, molds, animal dander, and insects. The allergen can be a polypeptide having an amino acid sequence at least 60%, 70%, 80%, 85%, 90%, 95% or 99% identical to SEQ ID NO:1, 2, or 3. The allergen can be Der p 1, Der p 2, and Der p 5 proteins.

Promoters that can be used to express the allergen include $\alpha$-lactalbumin, $\beta$-lactalbumin, whey acid protein, alpha, beta, gamma or kappa casein. The promoter can also be indirectly controlled to direct expression of the coding sequence in a mammary cell. For example, the promoter can be an inducible promoter, e.g., tetracyclin-activated promoter. A tetracyclin-regulated transcription factor can be selectively expressed in a mammary cell to express the coding sequence, e.g., when tetracyclin is also present. The nucleic acid sequence can be integrated in a chromosome.

The genetic composition of the transgenic mammal can include at least a second nucleic acid sequence, e.g., encoding a second allergen for expression in a mammary cell.

In some cases, the transgenic mammal can produce at least 100 mg, 500 mg, 1 g, 10 g, or 20 g or allergen / L of milk.

The transgenic mammal can be male or female. In the case of a male transgenic mammal, the nucleic acid is capable of directing expression of the allergen in a mammary cell of the female progeny of the male transgenic mammal. In the case of a female transgenic mammal, the nucleic acid is capable of directing expression of the allergen in a mammary cell of the female transgenic mammal or in a mammary cell of a female progeny of the female transgenic mammal.

The invention also features a mammalian germ cell, e.g., an egg or sperm cell, the genetic composition of which includes a nucleic acid sequence described herein.

The invention also features milk that contains a non-milk, heterologous allergen and a casein, i.e., an endogenous casein. The milk can also contain the allergen in a sufficient amount to reduce airway inflammation and hyperreactivity in a subject. The milk can also contain at least a second allergen. The casein can be, e.g., a naturally occurring casein of bovine or caprine origin, i.e., the milk from a cow or a goat. The milk can be obtained from a transgenic mammal described herein or a non-transgenic mammal in the case wherein the allergen polypeptide is added to the milk as an isolated polypeptide composition.

The invention also encompasses a method of decreasing the production of IgE in a subject exposed to an allergen. That method includes administering milk that contains a non-milk, heterologous allergen in a sufficient amount so that a subject who orally ingests the milk will become tolerant to the allergen. Thus, the subject will experience suppression of allergen-specific IgE production when the subject is subsequently exposed to the allergen. In some implementations the subject lacks a completely developed immune system, e.g., the subject is a neonate or infant. The invention features similar methods for reducing the broncopulmonary congestion in a subject.

In another aspect, the invention features, a method for providing a preparation which includes a non-milk allergen from the milk of a transgenic mammal including: obtaining milk from a transgenic mammal having introduced into its germline a non-milk allergen protein-coding sequence operatively linked to a promoter sequence that result in the expression of the protein-coding sequence in mammary gland epithelial cells, thereby secreting the non-milk allergen in the milk of the mammal to provide the preparation. The method can further include isolating the allergen from the milk or processing the milk, e.g., pasteurizing, condensing, or drying.

An "allergen" is defined as a substance that causes a Type I intermediate hypersensitivity reactions. An "aeroallergen" is defined as having at least the following characteristics: specific antigenic groupings that evoke active reaginic responses, and ambient exposure levels to which can lead to overt tissue changes in sensitive subjects. Aeroallergens are airborne particles that can cause respiratory, cutaneous, or conjunctival allergy. The water-soluble portion of ragweed pollen, for example affects the respiratory and conjunctival mucosa, and the lipid-soluble allergens of ragweed pollen can cause a typical contact dermatitis on exposed skin.

An "isolated nucleic acid" is a nucleic acid which has a non-naturally occurring sequence, or which has the sequence of part or all of a naturally occurring gene but is free of the genes that flank the naturally occurring gene of interest in the genome of the organism in which the gene of interest naturally occurs. The term therefore includes a recombinant DNA incorporated into a vector, into an autonomously replicating plasmid or virus, or into the genomic DNA of a prokaryote or eukaryote.

"Homology", or "percent identical," as used herein, refers to the sequence similarity between two polypeptide molecules or between two nucleic acid molecules. When a position in the first sequence is occupied by the same amino acid residue or nucleotide as the corresponding position in the second sequence, then the molecules are homologous at that position (i.e., as used herein amino acid or nucleic acid "homology" is equivalent to amino acid or nucleic acid "identity"). The percent homology between the two sequences is a function of the number of identical positions shared by the sequences (i.e., % homology = number of identical positions/total number of positions x 100). Generally, a comparison is made when two sequences are aligned to give maximum homology or sequence identity.

The comparison of sequences and determination of percent homology between two sequences can be accomplished using a mathematical algorithm. A preferred, nonlimiting example of a mathematical algorithm utilized for the comparison of sequences is the algorithm of Karlin and Altschul (1990) *Proc. Natl. Acad. Sci.* USA 87:2264-68, modified as in Karlin and Altschul (1993) *Proc. Natl. Acad. Sci.* USA 90:5873-77. Such an algorithm is incorporated into the NBLAST and XBLAST programs (version 2.0) of Altschul, et al. (1990) *J Mol. Biol.* 215:403-10. BLAST nucleotide searches can be performed with the NBLAST program, score = 100, wordlength = 12 to obtain nucleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilized as described in Altschul et al., (1997) *Nucleic Acids Res.* 25(17):3389-3402. When utilizing BLAST and Gapped BLAST programs, the default parameters of the respective programs (e.g., XBLAST and NBLAST) can be used. See http://www.ncbi.nlm.nih.gov.

By "hybridizes under stringent conditions" is meant specific and non-covalent equilibrium binding by base-pairing to an immobilized reference nucleic acid in a hybridization solution containing 0.2 X SSC (1.75 g/l NaCl, 0.88 g/l $Na_3$citrate$\cdot$2$H_2$O; pH 7.0) and 0.1% (w/v) sodium dodecylsulfate at 68°C. Washings, if any are required to achieve equilibrium, are carried out with the hybridization solution.

"Operably linked" is defined as the relationship between a gene and a regulatory sequence connected in such a way as to permit gene expression when the appropriate molecules (e.g., transcriptional activator proteins) are bound to the regulatory sequence(s).

A "promoter" is a nucleotide sequence that is capable of directing transcription in at least one context, e.g., when it is operably linked to a heterologous sequence in a cell. In other words, a promoter can exist without downstream sequences to transcribe, so long as the promoter sequence can direct transcription when placed upstream of a heterologous sequence in a different context.

As used herein, the term "subject" is intended to include human and non-human animals. The subject is typically a person, e.g., a patient having an allergic disorder.

"Tolerance" is defined as the state in which a mammal does not have a hypersensitive immune reaction upon exposure to an allergen or allergens.

As used herein, the term "transgene" means a DNA sequence that includes one or more selected DNAs, which is partly or entirely heterologous, i.e., foreign, to the transgenic animal, or homologous to an endogenous gene of the transgenic animal, but which is designed to be inserted into the animal's genome at a location which differs from that of the natural gene. A transgene includes one or more promoters and any other DNA, such as introns, necessary for expression of the selected DNA, all operably linked to the selected DNA, and may include an enhancer sequence.

"Transgenic" is defined as any cell which includes a DNA sequence which is inserted by artifice into a cell and becomes part of the genome of the organism which develops from that cell.

As used herein, the term "transgenic mammal" means a mammal which includes a transgene that is inserted into an embryonal cell and becomes a part of the genome of at least some of the cells of the mammal which develops from that cell, or an offspring of such a mammal. All mammals except humans are included in the invention. Examples of mammals include cows, sheep, goats, oxen, llamas, camels, horses, pigs, rabbits, and rodents such as guinea pigs, hamsters, rats, gerbils, and mice.

A "milk" or "milk composition" includes the secretions of the mammary gland of a mammal as well as any derivative thereof, e.g., a dried composition such as dried milk, condensed, and so forth.

Allergic disorders treatable by the invention include, without limitation, rhinitis, sinusitis, asthma, hypersensitive pneumonia, extrinsic allergic alveolitis, conjunctivitis, urticaria, eczema, dermatitis, anaphylaxis, angioedema, allergic and migraine headache, and certain gastrointestinal disorders in which IgE-mediated allergy are involved.

Other features or advantages of the present invention will be apparent from the following detailed description, and also from the claims.

Brief Description of the Drawings

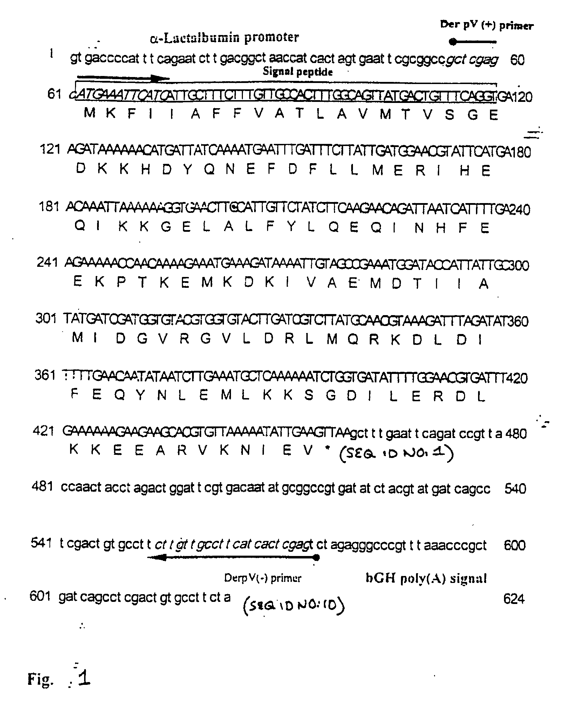

Fig. 1 shows the sequence of a transgene that includes the Der p 5 cDNA.

Detailed Description of the Invention

The invention relates to transgenic milk that can be therapeutically used to treat allergic disorders.

Using mammary gland-specific promoters, a wide range of proteins of biopharmaceutical interest have been expressed in rodents, pigs, and dairy animals (Echeland (1996) *Current Opinion in Biotechnology* 7:536; Houdebine (2000) *Transgenic Research* 9:505; and Meade *et al.* (1999) Chapter 14 in *Gene Expression Systems: Using Nature for the Art of Expression* Academic Press, page 399). An expression vector, comprising a gene encoding the target protein of interest fused to a milk promoter gene, is introduced by microinjection into the pronucleus of a one-cell embryo. Upon germ line integration and expression, the transgene becomes a dominant Mendelian genetic characteristic that is inherited by the progeny of the founder animal.

The transgenic offspring express the target protein, resulting in milk containing the target protein in as high as gram per liter quantities. Most frequently, the target protein takes the form of a soluble whey protein. Mammalian mammary epithelial cells have the capacity to carry out complex protein synthesis with a variety of posttranslational modifications and folding. The milk of transgenic livestock presents a starting material from which human diagnostic or pharmaceutically-active and therapeutic proteins may be purified using established technologies.

Manipulation of a milk composition by transgenesis has mainly focused on the mammary gland as a bioreactor to produce pharmaceuticals that are subsequently purified. The present improvement however, harnesses the gland's machinery to produce therapeutic milk that itself benefits its consumers. Thus, the time and cost of protein purification is completely avoided. Furthermore, since the therapy is contained in milk, it can be delivered so that the subject is completely unaware of its presence.

The specific allergen to be expressed for treating a particular allergy will of course depend on whether the undesirable immune response is targeted towards a protein allergen. Having identified a protein allergen that triggers the response, the skilled artisan can clone into a milk-specific expression vector a nucleotide sequence encoding the allergen. This expression vector is then used to generate a transgenic mammal and to obtain female individuals. The milk of such females is administered to an individual to ameliorate or prevent a subsequent symptom (e.g., skin inflammation or bronchopulmonary inflammation) characteristic of an allergy. Experimental protocols for evaluating and quantifying airway hyperreactivity is described below. Other methods of evaluating bronchopulmonary inflammation was well known in the art. Allergic asthma is characterized by both airway hyperreactivity and inflammation. Airway hyperreactivity can be measured by the changes of pulmonary test (invasive or non-invasive methods). Airway inflammation can be measured by the infiltration of inflammatory cells in the airway, especially eosinophils and neutrophils, and by changes in pathology.

For a further discussion of allergens and their physiological effects, see Aas et al. (1978) *Allergy* 33:3,; Goldin et al. (1998) *Br. J Nut.* 80:S203-S207; and Kailasapathy et al. (2000) *J. Immunol. Cell Biol.* 78:80-88.

Generally, allergens are operably linked to a milk promoter in a nucleic acid construct in order to provide the allergen in form for oral immunotherapy. The construct also includes a signal sequence for secretion of the allergen by a mammary epithelial cell, and other sequences such as a transcriptional terminator, an intron, a 5' untranslated region, or a polyadenylation site. The construct can include additional markers to facilitate transformation of a mammalian cell, e.g., markers for positive and/or negative selection.

The construct is then introduced into a cell, e.g., a fibroblast or an embryonic stem cell, such that the construct is integrated in the genome of the cell. For example, the construct is microinjected into the pronuclei of mammal embryos which are then transferred into pseudo-pregnant females. Using PCR screening and Southern blot hybridization, offspring with the transgene are identified. After mating with normal mammals, F1 female offspring are produced. The transgenic milk that these female offspring produce can then be used to treat allergic disorders. Furthermore, the transgenic milk can be used in primary prevention, i.e. it is given to infants that have a high risk of being affected by allergic disorders because one or more of their parents is affected by allergic disorders.

Transgenic animals can also be used to propagate the transgene, e.g., to form a herd of animals. Milk can be routinely obtained from female individuals of such a herd use art-known methods.

General methods for designing the construct can include the following details for specific features:

Allergens

Any polypeptide allergen or allergens can be delivered using the methods provided herein. For example, the polypeptide allergen can be a polypeptide encoded by a plant, fungus, or animal. Plant allergens are frequent in pollen, sap, leaves, and plant toxins (e.g., secretions of poison ivy and so forth). Fungal allergens include polypeptides produced by molds, *Aspergillus*, and others. Animal allergens include, e.g., polypeptides produced by insects, e.g., fecal allergens of dust mites, and mammals, e.g., cat dander.

A nucleic acid sequence encoding an allergen can be identified by any of a variety of methods. A cDNA expression library can be screened using IgE from an allergic individual (Lin *et al.* (1994) *J Allergy Clin Immunol* 94: 989-96). Positive clones are identified and sequenced. The coding region can be identified and translated to determine the polypeptide sequence for the allergen. The coding region can be used to express the allergen in a nucleic acid described herein.

Particular examples of polypeptide allergens include the Der p allergens of *Dermatophagoides pteronyssinus.* The Der p 5 polypeptide sequence is recited as follows:

MKFIIAFFVATLAVMTVSGEDKKHDYQNEFDFLLMERIHEQIKKGELALF YLQEQINHFEEKPTKEMKDKIVAEMDTIIAMIDGVRGVLDRLMQRKDLDIFEQ YNLEMAKKSGDILERDLKKEEARVKKIEV (SEQ ID NO:1). See also Lin *et al.* (1994) *J Allergy Clin Immunol* 94:989-96.

The Der p 1 polypeptide sequence is recited as follows:

MKIVLAIASLLALSAVYARPSSIKTFEEYKKAFNKSYATFEDEEAARKNF LESVKYVQSNGGAINHLSDLSLDE-FKNRFLMSAEAFEHLKTQFDLNAETNACSI NGNAPAEIDLRQMRTVTPIRMQGGCGSCWAFSGVAATESAYLAYRN-QSLDLA EQELVDCASQHGCHGDTIPRGIEYIQHNGVVQESYYRYVAREQSCRRPNAQRF GISNYCQIYPPNVNKIREALAQTHSAIAVIIGIKDLDAFRHYDGRTIIQRDNGYQP NYHAVNIVGYSNAQGVDYWIVRN-SWDTNWGDNGYGYFAANIDLMMIEEYPY VVIL (SEQ ID NO:2).

The Der p 2 polypeptide sequence is recited as follows:

MMYKILCLSLLVAAVARDQVDVKDCANHEIKKVLVPGCHGSEPCIIHRG KPFQLEAVFEANQNTKTAKIEIKASIDGLEVDVPGIDPNACHYMKCPLVKGQQ YDIKYTWNVPKIAPKSENVVVTVKVMGDDGVLACAIATHAKIRD (SEQ ID NO:3).

Milk Specific Promoters

A variety of transcriptional promoters that preferentially activate transcription in mammary epithelial cells are available. These include the promoters that control the genes encoding milk proteins such as caseins, beta lactoglobulin (Clark et al., (1989) Bio/Technology 7: 487-492), whey acid protein (Gordon et al. (1987) Bio/Technology 5: 1183-1187), and lactalbumin (Soulier et al., (1992) FEBS Letts. 297: 13). Casein promoters may be derived from the alpha, beta, gamma or kappa casein genes of any mammalian species; a preferred promoter is derived from the goat beta casein gene (DiTullio, (1992) Bio/Technology 10:74-77).

The DNA sequences of many these promoters are available, e.g., in GenBank and in scientific publications such as Richards et al., (1981) *J Biol. Chem.* 256, 526-532 (α-lactalbumin rat); Campbell et al., (1984) *Nucleic Acids Res.* 12:8685-8697 (rat WAP); Jones et al., (1985) *J. Biol. Chem.* 260:7042-7050 (rat β-casein); Yu-Lee & Rosen (1983) *J. Biol. Chem.* 258:10794-10804 (rat γ-casein); Hall, (1987) *Biochem. J* 242, 735-742 (α-lactalbumin human); Stewart, (1984) *Nucleic Acids Res.* 12, 389 (bovine αs1 and κ casein cDNAs); Gorodetsky et al., (1988) *Gene* 66:87-96 (bovine β casein); Alexander et al., *Eur. J Biochem.* 178:395-401 (1988) (bovine κ casein); Brignon et al., (1977) *FEBS Lett.* 188, 48-55 (bovine αS2 casein); Jamieson et al., (1987) *Gene* 61, 85-90, Ivanov et al., (1988) *Biol. Chem. Hoppe-Seyler* 369, 425-429, Alexander et al., (1989) *Nucleic Acids Res*. 17, 6739 (bovine β lactoglobulin); Vilotte et al., (1987) *Biochimie* 69, 609-620 (bovine α-lactalbumin). See also Mercier & Vilotte, (1993) *J Dairy Sci.* 76, 3079-3098for a general review. For additional regulatory control or stringency, other regulatory sequences, e.g., adjacent sequences, can be obtained from these genes or from homologous genes of other mammals using these promoter sequence as probes to screen genomic libraries.

Signal Sequences

The nucleic acid contruct can also include a signal sequences, particularly a signal sequences of a milk specific gene. The milk-specific signal sequence can be the signal sequence which naturally occurs with the selected milk-specific promoter used in the construct, which are described below. For example, signal sequences from genes coding for caseins, e.g., alpha, beta, gamma or kappa caseins, beta lactoglobulin, whey acid protein, and lactalbumin can be used.

Transgenesis

A transgenic mammal can also be produced by nuclear transfer of a transgenic cell line into which a nucleic acid of interest, e.g., a nucleic acid which encodes allergen, into an embryonic stem cell. Methods for the introduction of foreign DNA into mammals include microinjection injection and nuclear transplantation methods. For example, protocols for producing transgenic goats, pigs, rats, cows, and sheep are available. See, e.g., Ebert et al. (1994) *Bio/Technology* 12:699; PCT Application WO 98/30683; White and Yannoutsos, *Current Topics in Complement Research: 64th Forum in Immunology*, pp. 88-94; US Patent No. 5,523,226; US Patent No. 5,573,933; PCT Application WO93/25071; PCT Application WO95/04744; Bader and Ganten, *Clinical and Experimental Pharmacology and Physiology*, Supp. 3:S81-S87, 199; U.S. Patent No: 5,741,957, PCT Application WO 98/30683; *Transgenic Animal Technology, A Handbook,* 1994, ed., Carl A. Pinkert, Academic Press, Inc; PCT Publication WO 97/07669; and *Transgenic Animal Technology, A Handbook*, 1994, ed., Carl A. Pinkert, Academic Press, Inc.

A construct can be introduced into a cell via conventional transformation or transfection techniques including calcium phosphate or calcium chloride coprecipitation, DEAE-dextrane-mediated transfection, lipofection, or electroporation (see, e.g., Sambrook et al., *Molecular Cloning: A Laboratory Manuel, 2nd ed., Cold Spring Harbor Laboratory,* (Cold Spring Harbor Laboratory Press, Cold Spring Harbor, NY, 1989)).

Without further elaboration, one skilled in the art can, based on the above disclosure and the efficacious treatment using transgenic milk discussed below, utilize the present invention to its fullest extent. The following example is to be construed as merely illustrative of how one skilled in the art can produce and use transgenic animals to synthesize therapeutic milk, and is not limitative of the remainder of the disclosure in any way. All publications cited in this disclosure are hereby incorporated by reference.

Example

Methods and Material

*Transgene Construction and Transgenic Mice Production.* A 2.0-kb promoter sequence of bovine alpha-lactalbumin (αLA) was generated by PCR amplification using Holstein cow DNA as the template and pαLA-F: 5'-TCGCG TAGAATCGATTCATGT-3' (SEQ ID NO:4)/ pαLA-R:5'-TACGCGTAGCCTGGGTGGCAT-3' (SEQ ID NO:5) as the primer set. This PCR product containing entire αLA promoter sequence was inserted into the T-protruding pCR3 vector (Invitrogene, San Diego, CA). The resulting 7.0-kb plasmid was digested with *Xho*I and treated with calf intestinal phosphatase (Boehringer Mannheim, Indianapolis, IN). The Der p 5 cDNA was amplified using a Der p 5(+)/Der p 5(-) primer set (5'-GCTCGAGCATGAAATTCATC -3' (SEQ ID NO:6)and 5'-ACTCGAGTGATGAAGGCAACAAG -3' (SEQ ID NO: 7) respectively) from a pGST/Der p 5 plasmid containing the intact mite Der p 5 coding sequence and the PCR product was also digested with *Xho*I and further ligated into the pCR3-αLA vector. Several clones were screened, and clones containing the insert in the correct orientation were selected. The in frame sequence from the αLA promoter through the Der p 5 junction was determined using the Dye Terminator sequencing system (Applied Biosystems Inc., Foster, CA). The 2.8-kb transgene consisting of 2.0-kb bovine αLA promoter, 0.5-kb Der p 5 cDNA, and 0.3-kb bovine GH gene polyadenosine signal sequence was separated from plasmid pCR-aLA/Der p 5-12 using *BamH*I and *Bbs*I digestions and purified for microinjection on the twice $CsCl_2$ gradient ultra-centrifugation.

The purified transgene, see, e.g., Fig. 1, was microinjected into the male pronuclei of fertilized eggs from superovulated female mice of the outbreed ICR strain and transferred to recipient pseudo-pregnant females as described in Chen *et al.*, Science 1994;265:1237-40. The resulting pups were rapidly screened for the transgene by PCR amplification of tail DNA. Results were further confirmed by Southern blot analysis.

*PCR Screening and Southern Blot Hybridization.* PCR rapid screening was performed using one set of primer, pαLA-124(+): 5'-CTCTCTTGTCATCCTCTTCC-3' (SEQ ID NO:8) and pDer p 5(-): 5'-ACTCGAGTGATGAAGG CAAGAAG-3' (SEQ ID NO:9), which defined a 580-bp region spanning the αLA promoter and Der p 5 cDNA junctional sequence.

Ten micrograms of genomic DNA were digested with StuI and XbaI restriction enzymes individually at 37°C overnight, electrophoresed on a 0.8% agarose gel, and transferred to a Durose membrane (Stratagene, La Jolla, CA). An XhoI fragment of Der p 5-specific cDNA (0.5-kb) was used as a radioactive probe to hybridize the membrane. Blots were subjected to autoradiography for one week at -20°C freezer.

*Immunohistochemistry Assay of Mammary Gland Tissue in Lactating Mice.* Freshly dissected mammary glands from transgenic and non-transgenic mice in the D15 lactating stage were fixed in paraformaldehyde following the protocol of Sassoon et al. (1988) *Development* 104: 155-164.

Tissue sections (5-10 μm) were stained with hematoxylin and eosin B red dyes and were photographed under a light microscope (Axiovert 135; Carl Zeiss, Germany). Immunohistochemical analysis was conducted according to Gruskin-Lerner and Trinkaus-Randall (1991) *Curr Eye Res* 10: 75-85. A rabbit anti-mite Der p 5 polyclonal antibody was diluted 1:30 (1% bovine serum albumin [BSA] in phosphate-buffered saline [PBS]) and 50 μl aliquots were incubated with either anti-Der p 5 or normal rabbit serum (NRS; 1: 10) for 30 min at 37 °C. After washing with 10 volumes of BSA-PBS buffer, the mammary gland slides were incubated with affinity purified goat anti-rabbit IgG antibody conjugated to fluorescein isothiocyanate (FITC; Boehringer-Mannheim, Germany) at a dilution of 1: 100 (1% BSA in PBS) for 30 min at 37 °C. After washing, the slides were observed under a Nikon Optiphot microscope (Nikon, Tokyo, Japan) equipped with epifluorescence optics.

*Immunoblot Analysis of Transgenic Mice Milk.* Milk was collected from lactating females as previously described (Simons *et al.* (1987) *Nature* 328:530-532) and analyzed using SDS-polyacrylamide gel electrophoresis (SDS-PAGE). Ten- to twenty-fold dilutions of the collected milk, in 75 mM Tris-HCl buffer at pH 6.8, from different lactation periods were diluted in SDS-PAGE sample buffer with 5% 2-mercaptoethanol and electrophoresed on 7.5% gel. To estimate production levels, a purified recombinant Der p 5 standard was diluted to 10 μg/ml in normal mouse milk and electrophoresed alongside the transgenic milk samples. Proteins were electrotransferred from the gel to a PVDF membrane (NEN Life Science Products, Boston, MA). The blots were probed with primary antibodies recognizing Der p 5 at 2 to 10 μg/ml and washed with phosphate-buffered saline containing 0.1% Tween-20 (PBS-T). Blots were reacted with horseradish peroxidase (HRP)-conjugated second antibodies at 0.2 μg/ml. The blot was then developed with the chemiluminescent ECL™ detection system (Amersham, UK) and exposure to x-ray film. Band intensities were compared by densitometry.

*Animals and Study Protocol.* Female LCR, aged 21 days, were obtained from the animal-breeding center of the College of Medicine, National Taiwan University (originated from The Jackson Laboratory, Bar Harbor, ME.), were divided into 3 groups for each experiment (Table 1). Animals were fed with transgenic milk containing Der p 5 or milk only for 4 weeks. Then animals were actively sensitized by intraperitoneal injection of 10 μg of recombinant Der p 5 that was purified by known techniques. Mice received oral feeding of LA-gm or ST-gm LAB $10^9$ for 3 days per week for 2 weeks. Twenty-one days after the sensitization, animals were exposed to an aerosol of either 0.1% of Der p 5-Glutathion S-transferase fusion protein for 20 min or PBS. Eight hours after inhalational challenge, pulmonary resistance was measured for 50 min, and the bronchoaveolar lavage fluids (BALF) and sera were collected.

*Non Invasive Method for the Determination of Airway Responsiveness.* Using barometric whole body plethysmography (WBP) (Buxco, Troy, NY, USA), response to inhaled methacholine in conscious, unrestrained mice were measured using conventional methods. Before taking readings, the box was calibrated with a rapid injection of 1 ml of air into the main chamber to obtain the 1 mv signal from the WBP. Inspiration and expiration were recorded by establishing start-inspiration and end-inspiration, as the box pressure/time curve crossed the zero point. Start of an inspiration was determined by extrapolating from a straight line drawn from two levels of the rising inspiratory phase of the box pressure signal. Time of inspiration (T1) was defined as the time from the start of inspiration to the end of inspiration; time of expiration (TE) as the time from the end of inspiration to the start of the next inspiration. The maximum box pressure signal occurring during one breath in a negative or positive direction was defined as peak inspiratory pressure (*PIP*) or peak expiratory pressure (*PEP*), respectively. Recordings of every 10 breaths were extrapolated to define the respiratory rate in breaths per minute. The relaxation time (Tr) was defined as the time until a 36% of the total expiratory pressure signal (area under the box pressure signal in expiration) occurred. This served as a correlate to the time constant of the decay of the volume signal to 36% decay of the peak volume in passive expiration. *Pause* and *Penh* were defined and calculated by the follwing formulae:

$$Pause = (Te - Tr) / Tr$$

$$Penh\ (Enhanced\ Pause) = (PEP / PIP) \times Pause$$

As an index of airway responsiveness, increases in enhanced pause (*Penh*) were measured. Mice were obtained and averaged for 3 min. Aerosized saline, followed by increasing concentrations of methacholine (ranging from 1-100 mg/ml), was nebulized for 3 min, and readings were taken and averaged for 3 min after each nebulization. Airway responsiveness was expressed as the *Penh* per dose methacholine.

*Statistical Analysis.* To assess changes of pulmonary resistance, cytokine concentration, IgE, IgG level, and cells in the BALF after Der p 5 challenge, repeated measures for ANOVA were performed to compare the differences among groups. Following analysis of variance, Duncan multiple range test was used to differentiate differences between experimental and control groups. A $p < 0.05$ was used to indicate statistical significant difference.

Results

*Suppression of Airway Hyperreactivity (AHR) In Vivo.* Oral feeding with transgenic milk into animals was observed to suppress the allergen-induced AHR. Airway responsiveness to aerosolized methacholine was measured *in vivo* 8 hours after last inhalational challenge in conscious, unrestrained mice. For all mice, a complete dose-response curve to methacholine raging from 1 to 100 mg/ml was constructed. In all groups of mice, no significant differences were observed in the basal Penh and saline aerosol-induced Penh values. In mock (Group C)-treated mice, Der p 5 inhalational challenge induced a significant increase in airway responsiveness to methacholine compared with the baseline. In contrast, treatment with transgenic milk (Group A) to Der p 5 sensitized mice unexpectedly inhibited the AHR at doses ranging from 10 to 100 mg/ml methacholine.

Table 1.

| Characteristics of three experimental groups and procedures performed for each group | | | |
|---|---|---|---|
| Group | Treatment[a] | Sensitiztion[b] | Inhalational challenge[c] |
| A Milk | containing Der p 5 | Der p 5 | + |
| B | Milk containing Der p 5 | Alumin hydroxide | + |
| C | milk | Der p 5 | + |

a: feeding with milk containing Der p 5 for 28 days

b: injected intraperitioneally with 10 ug rDer p 5 and 4 mg Al(OH)3

c: inhatonal challenge with Der p 5 0.1% for 15 minutes

In Group C, the septa are infiltrated with massive inflammatory cells (lymphocytes, macrophages and also some eosinophils) especially around the bronchioles and capillaries. The lung inflammation is typified by the presence of giant cells. In the space of alveoli are filled of mucous secretion. The inflammatory level was severe.

On the other hand, the histopathological study in the lungs of group A revealed that only some inflammatory cells infiltrated the bronchioles and capillaries. The septa did not thicken and the space of alveoli was clear. The inflammatory level was correspondingly as mild. These observations also demonstrate that treatment with transgenic milk containing Der p 5 suppressed the development of Der p 5-induced specific airway inflammation, compared to animal treated with milk only.

Other Embodiments

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A non-human transgenic mammal, the genetic composition of which comprises:

    A nucleic acid that includes (1) a coding sequence that encodes an allergen and (2) a heterologous promoter operably linked to the coding sequence, wherein the heterologous promoter directs expression of the allergen in a mammary cell of the animal or a female progeny thereof.

2. The transgenic mammal of claim 1, wherein the allergen is of a dust mite, the dust mite preferably being *Dermatophagoides pteronyssinus* or *Dermatophagoides farinae*.

3. The transgenic mammal of claim 1 or 2, wherein the allergen has an amino acid sequence at least 70% identical to SEQ ID NO: 1, 2 or 3.

4. The transgenic mammal of claim 3, wherein the allergen has an amino acid sequence that is identical to SEQ ID NO: 1, 2 or 3.

5. The transgenic mammal of any of claims 1 to 4, wherein the promoter is a promoter for a casein or lactalbumin, preferably α-lactalbumin promoter.

6. The transgenic mammal of any preceding claim, wherein the mammal is a cow, goat or sheep; and/or wherein the allergen is a protein allergen.

7. A milk composition comprising:

    a heterologous, non-milk allergen; and
    a casein, for use as a vaccine.

8. The milk composition of claim 7, wherein the allergen is of an insect, preferably of a dust mite.

9. The milk composition of claim 8, wherein the dust mite is *Dermatophagoides pteronyssinus* or *Dermatophagoides farinae.*

10. The milk composition of claim 9, wherein the allergen is Der p 5, Der p 1, or Der p 2.

11. The milk composition of any of claims 7 to 10, wherein the casein is bovine or caprine; and/or, wherein the composition is dried; and/or wherein the allergen is a protein allergen.

12. Use of the milk composition of any of claims 7 to 11 in the preparation of a medicament for the treatment of airway inflamation and hyperactivity in a human or animal patient.

13. Use of the milk composition of any of claim 7 to 11 in the preparation of a medicament for decreasing the production of IgE in a human or animal patient exposed to an allergen.

14. A nucleic acid comprising (1) a coding sequence that encodes an allergen; and (2) a heterologous promoter operably linked to the coding sequence wherein the heterologous promoter directs expression of the allergen in a mammary cell.

15. A nucleic acid consisting of:

(1) a nucleic acid sequence identical to SEQ ID NO: 1, 2 or 3.
(2) a heterologous promoter operably linked to the coding wherein the heterologous promoter directs expression of the allergen in a mammary cell.

**16.** The nucleic acid of claim 14 or 15, wherein the allergen is of a dust mite allergen, preferably the dust mite being *Dermatophagoides pteronyssinus* or *Dermaptophgoides farinae.*

**17.** The nucleic acid of claim 14, 15 or 16, wherein the allergen has an amino acid sequence at least 70% identical to SEQ ID NO: 1, 2 or 3.

**18.** The nucleic acid of claim 14 to 17, wherein the allergen has an amino acid sequence that is identical to SEQ ID NO: 1, 2 or 3.

**19.** The nucleic acid of any of claims 14 to 18, wherein the promoter is a promoter for a casein or lactalbumin, preferably $\alpha$-lactalbumin; and/or, wherein the allergen is a protein allergen.

**20.** A mammalian germ cell of the transgenic mammal of any of claims 1 to 6, wherein the germ cell includes the nucleic acid.

α-Lactalbumin promoter             Der pV (+) primer

```
1  gt gaccccat t t cagaat ct t gacggct aaccat cact agt gaat t cgcggcc gct cgag  60
                                         Signal peptide
61 cATGAAATTCATCATTGCTTTCTTTGTTGCCACTTTGGCAGTTATGACTGTTTCAGGTGA 120
     M  K  F  I  I  A  F  F  V  A  T  L  A  V  M  T  V  S  G  E

121 AGATAAAAAACATGATTATCAAAATGAATTTGATTTCTTATTGATGGAACGTATTCATGA 180
     D  K  K  H  D  Y  Q  N  E  F  D  F  L  L  M  E  R  I  H  E

181 ACAAATTAAAAAAGGTGAACTTGCATTGTTCTATCTTCAAGAACAGATTAATCATTTTGA 240
     Q  I  K  K  G  E  L  A  L  F  Y  L  Q  E  Q  I  N  H  F  E

241 AGAAAAACCAACAAAAGAAATGAAAGATAAAATTGTAGCCGAAATGGATACCATTATTGC 300
     E  K  P  T  K  E  M  K  D  K  I  V  A  E  M  D  T  I  I  A

301 TATGATCGATGGTGTACGTGGTGTACTTGATCGTCTTATGCAACGTAAAGATTTAGATAT 360
     M  I  D  G  V  R  G  V  L  D  R  L  M  Q  R  K  D  L  D  I

361 TTTTGAACAATATAATCTTGAAATGCTCAAAAAATCTGGTGATATTTTGGAACGTGATTT 420
     F  E  Q  Y  N  L  E  M  L  K  K  S  G  D  I  L  E  R  D  L

421 GAAAAAAGAAGAAGCACGTGTTAAAAATATTGAAGTTAAgct t t gaat t cagat ccgt t a  480
     K  K  E  E  A  R  V  K  N  I  E  V  *  (SEQ ID NO: 1)

481 ccaact acct agact ggat t cgt gacaat at gcggccgt gat at ct acgt at gat cagcc  540

541 t cgact gt gcct t ct t gt t gcct t cat cact cgagt ct agagggcccgt t t aaacccgct  600
            DerpV(-) primer          hGH poly(A) signal
601 gat cagcct cgact gt gcct t ct a  (SEQ ID NO: 10)            624
```

Fig. 1